# EUROPEAN PATENT APPLICATION

(11) **EP 3 453 722 A1**
(43) Date of publication of application: **13.03.2019**
(21) Application number: 17189661.6
(22) Date of filing: 06.09.2017
(51) Int. Cl.: C07K 14/65, A61K 38/30, C12N 9/10

(54) **PHARMACEUTICALS AND DEVICES FOR THE COVALENT IMMOBILIZATION TO THE EXTRACELLULAR MATRIX BY TRANSGLUTAMINASE**

(71) Applicant: Julius-Maximilians-Universität Würzburg, 97070 Würzburg (DE)
(72) Inventor: Braun, Alexandra, 97218 Gerbrunn (DE); Meinel, Lorenz, 97082 Würzburg (DE); Lühmann, Tessa, 97070 Würzburg (DE)
(74) Representative: Engelhard, Markus

(57) **Abstract**

The present invention relates to compounds and their use in the treatment of lesions, in tissue regeneration and/or tissue engineering. The compounds act as substrates for enzymes having transglutaminase activity and are suitable for their immobilization on extracellular substrates or matrices.

## Description

The present invention relates to compounds and their use in the treatment of lesions, in tissue regeneration and/or tissue engineering. The compounds act as substrates for enzymes having transglutaminase activity and are suitable for their immobilization and/or attached therapeutic or diagnostic molecules on extracellular matrix (ECM) or synthetic ECM-derived materials.

### Background of the invention:

In natural situations, growth factors (GFs) such as transforming growth factor beta (TGF-β), vascular endothelial growth factor (VEGF), fibroblast growth factor 2 (FGF-2) and platelet-derived growth factor (PDGF) [1] key signaling molecules regulating tissue repair and regeneration [1] - are present bound to the ECM, but therapeutic use of such growth factors has focused on application in soluble forms. Thus, GFs are quickly cleared from the treated area upon delivery. Physiologically, GFs are not only secreted by cells, but they are also sequestered locally by the ECM. The ECM then controls the spatiotemporal release of GFs and modulates their intracellular signaling, which makes them highly effective at very low dose and allows proper tissue morphogenesis. In contrast, growth factors delivered in soluble forms underlie limited clinical translation due to safety issues and cost effectiveness and several studies highlight the complex biomolecular interactions between growth factors and ECM proteins dramatically altering the biology of the growth factor [2].

Attempting to transfer this natural strategy to highly potent growth factors and cytokines and retain them in the ECM, we propose an approach to modify them with a high-affinity transglutaminase substrate sequence derived from Insulin-like Growth Factor-I (IGF-I). Human IGF-I is a peptide hormone of 7.6 kDa playing an important role in tissue repair, regeneration and growth by stimulating anabolic and anti-apoptotic processes within tissues. Thus, IGF-I is a potential treatment option for muscular atrophy [3]. Recombinant human IGF-I (Mecasermin) has a reported half-life of 5.8 hours at doses of 0.12 mg/kg after subcutaneous injection, likely resulting from IGFBP binding upon administration [4]. The relatively short half-live and the importance of paracrine action of IGF-I have sparked interest in the development of parenteral IGF-I depot systems providing sustained localized delivery, e.g. from poly(D,L-lactide-co-glycolide) acid (PLGA) microspheres [5-7]. IGF-I consists of 70 amino acids and is structurally and functionally related to insulin but displays a much higher growth-promoting activity. Mature IGF-I protein consists of four domains named BCAD (in order from N- to C-terminus) and the precursor IGF-I protein also has an E peptide at the C terminus. Critical residues in domains A, B, and C are involved in receptor binding and activation as well as in binding of IGFBPs [9, 10]. Interestingly, the functional importance of the D-domain of IGF-I is still not completely understood as its removal does not reduce receptor binding and it is the only domain to remain exposed to antibodies when bound to IGF-IR or IGFBPs. Previous studies found, that IGF-I contains an intrinsic transglutaminase substrate domain and can therefore be modified exclusively at the lysine residue #68 within the D domain by factor Xllla or tissue transglutaminase [11]. The conjugation partner can be modified with a glutamine substrate (such as the sequence NQEQVSPL derived from α-2 plasmin inhibitor - one of the key molecules in controlling the degradation of fibrin), enabling covalent and site-specific conjugation to IGF-I. Factor XIIIa - a human transglutaminase activated from factor XIII by thrombin proteolysis during wound healing - is responsible for cross-linking fibrin monomers as an initial response to enclose the wound during injury. This reactivity was extensively examined in previous studies and was deployed for site-specific protein conjugation [12]. After activation by thrombin, activated FXIII (FXIIIa) catalyzes the formation of a covalent ε-(γ-glutamyl)-lysine isopeptide bond between a fibronectin glutamine residue and a lysine side chain of a peptide, protein, or small molecular probe. Transamidation by FXIIIa has already found application in the incorporation of exogenous peptides into fibrin gels, the controllable crosslinking in biological or synthetic hydrogels, and for surface functionalization [12-15].

Growth factors such as IGF-I, vascular endothelial growth factor (VEGF), fibroblast growth factor 2 (FGF-2) and bone morphogenetic protein 2 (BMP-2) have previously been covalently immobilized to biomaterials/ECM by conventional chemical conjugation approaches based on lysines or cysteines in the protein sequence, preferably by 1-Ethyl-3-(3-dimethylaminopropyl)carbodiimide (EDC)/N-hydroxysuccinimide (NHS) chemistry with the aim to (re-)engineer lost tissues [1]. A remarkable drawback of this EDC/NHS chemistry is that most biologic molecules carry both carboxyl and amino groups giving rise to intra-and/or intermolecular crosslinking and heterogeneity, which can compromise pharmacokinetics and limit efficacy. Additionally, due to differences in the location of the conjugation site, unspecific coupling might trigger protein aggregation, instability and reduced affinity to the target, potentially resulting in adverse side effects and impaired potency. Homogenous conjugates were produced by, e.g., introduction of unnatural functional groups for bio-orthogonal protein conjugation using chemical approaches, or by attaching recombinantly a 'tag' to the protein structure for recognition by a specific enzyme that catalyzes the cross-linking to a certain substrate.

This approach of using the D domain of IGF-I as anchor enables covalent immobilization of therapeutic or diagnostic molecules on ECM. The ECM is a highly dynamic microenvironment and provides mechanical stability, the possibility of cell migration by displaying cell-binding sites (for integrin receptors), the possibility of modulation cell processes (including proliferation and differentiation) and acts as a reservoir for growth factors. Many growth factors have the ability to bind to specific sites within the ECM and will thus first interact with the ECM before interaction with cell surface receptors. Once bound to the ECM their release depends on their binding affinity and the enzymatic turnover of the ECM. Fibronectin (Fn), a glycoprotein of the ECM, has a N-terminally located glutamine residue representing a natural transglutaminase substrate [16, 17]. Fn is an approximately 440 kDa extracellular matrix protein that is excreted by cells and plays major roles in cell adhesion, migration and growth factor storage. It is composed of a multimodular structure containing three types of repeating globular modules, which display a number of molecular recognition sites (fibrillogenesis, the RGD loop mediates cell adhesion and accumulates growth factors) [17]. The Fn N-terminal tail represents a suitable conjugation site preserving the accessibility of natural probes/cells to their target sites.

However, there is still a need for an improvement of the immobilization of polypeptides to matrices, wherein the polypeptide is capable of exerting a desired, e.g., a therapeutic or diagnostic activity. Many of the sequences previously proposed for immobilization of proteins or peptides using enzymatic ligation strategies lack selectivity leading to various byproducts with the need for complex purification, or result in low yield [12, 17].

### Short summary of the invention

The above need is fulfilled by the present invention, which relates, e.g., to the site-specific incorporation of therapeutic or diagnostic molecules, e.g., native or modified, such as truncated forms of IGF-I, or other growth factors or therapeutically active or diagnostic molecules into the extracellular matrix (ECM) protein fibronectin (Fn) by using the enzyme-catalyzed transamidation reaction mediated by human factor Xllla (activated plasma transglutaminase). Taking advantage of this natural principle, nature's physiological processes can be emulated during wound/lesion healing and tissue repair and provide local pools of, for example, IGF-I to facilitate tissue regeneration, intended, amongst others, for the treatment of wounds or lesions and the prevention of post-operative complications, or to facilitate tendinitis repair, bone regeneration, cartilage repair or muscle wasting. For other therapeutically active molecules that are linked to suitable components of the ECM (e.g., fibrinogen) the respective clinical indications that may be treated with such therapeutically active molecules correspond to the skilled person's knowledge.

The design of controlled release strategies for highly potent biologics, such as growth factors, to achieve temporal and spatial dose localization is an ongoing challenge. The ECM plays a fundamental role in coordinating growth factor signaling *in vivo,* by displaying and releasing them in a highly spatio-temporal controlled manner and also by modulating their intracellular signaling. The simultaneous application of IGF-I together with the cross-linking enzyme promotes local pools of IGF-I anchored onto the ECM, thereby enabling local accumulation and growth factor storage in the ECM, according to Nature's developed strategy.

Characterization by SDS-PAGE, Western Blotting, mass spectrometry, HPLC analysis and immobilization on ECM with subsequent antibody staining shows that the transamidation reaction is specific for fibronectin and for the amino acid residue K68 in IGF-I, and that the use of a truncated version ('D domain', consisting of the last 8 amino acids of IGF-I, SEQ ID NO: 1) is sufficient for recognition and cross-linking by human factor XIIIa. The coupling reaction is highly effective, follows very fast kinetics (< 2 min) and the bioactivity of ECM immobilized IGF-I is preserved.

When residue K6 in SEQ ID NO: 1 (corresponding to residue K68 in IGF-1) is replaced by arginine (as shown in SEQ ID NO: 12) IGF-1 cannot be directly bound to the ECM in a transglutaminase-mediated reaction. However, the incorporation of a bioresponsive linker into IGF-1, followed by the D' domain on the other hand allows the binding of such synthetic constructs to the ECM. Thereby, a tailored IGF-1 growth factor release (or the release of other therapeutic molecules or diagnostic molecules in similarly constructed synthetic compounds) can be achieved by integration of cleavable linkers responding to elevated protease concentrations during inflammation (e.g. matrix metalloproteinases [18]) or changes in external stimuli, such as a reduction in pH or elevated H₂O₂ concentrations [19]. The isolated D domain (SEQ ID NO: 1) can also be inserted into any other protein structure of interest to provide a chimeric or fusion polypeptide sequence (e.g, comprising a polypeptide such as fibroblast growth factor 2 (FGF-2) promoting anchoring onto the ECM, thereby enabling local accumulation and growth factor storage in the ECM, according to Nature's developed strategy.

### Detailed description of the invention

Before disclosing the subject-matter in greater detail, definitions of terms/expressions used herein are provided.

Unless otherwise defined, all technical and scientific terms used herein have the same meaning as commonly understood by one of ordinary skill in the art to which this invention belongs. Although methods and materials similar to those described herein can be used in the practice or testing of the present invention, suitable methods and materials are described below. In addition, the materials, methods, and examples are illustrative only and not intended to be limiting.

Each of the patents, applications and articles cited herein, and each document cited or referenced therein, including during the prosecution of any of the patents and/or applications cited herein ("patent cited documents"), and any manufacturer's instructions or catalogues for any products cited herein or mentioned in any of the references and in any of the patent cited documents, are hereby incorporated herein by reference. Documents incorporated by reference into this text or any teachings therein may be used in the practice of this invention. Documents incorporated by reference into this text are not admitted to be prior art. As used herein, the words "may" and "may be" are to be interpreted in an openended, non-restrictive manner. At minimum, "may" and "may be" are to be interpreted as definitively including structure or acts recited.

The articles "a" and "an" are used herein to refer to one or to more than one (i.e. to at least one) of the grammatical object of the article.

Throughout this specification, unless the context requires otherwise, the words "comprise", "comprises" and "comprising" will be understood to imply the inclusion of a stated step or element or group of steps or elements but not the exclusion of any other step or element or group of steps or elements.

Each of the respective terms "biologically active fragment" or "pharmaceutically active fragment" or "therapeutically active fragment" is meant a fragment of a full-length parent polypeptide which fragment retains the activity of the parent polypeptide. A biologically active fragment will therefore have, for example, at least 75% of the activity of the polypeptide according to SEQ ID NO: 1 using the same test conditions to quantify the activity, namely being suitable for incorporation into the extracellular matrix (ECM) or synthetic ECM-derived materials. In preferred embodiments, the fragments maintain the lysine residue at position 6 of SEQ ID NO: 1.

The terms "biologically active fragment" includes deletion variants and peptides comprising an polypeptide according to SEQ ID NO: 1, for example, of at least 4, at least 5, at least 6, at least 7, at least 8, at least 9, at least 10, at least 15, at least 20, and at least 25, least 30 contiguous amino acids, which comprise the above activities. Peptides of this type may be obtained through the application of standard recombinant protein expression techniques or synthesized using conventional liquid or solid phase peptide synthesis techniques. For example, reference may be made to solution synthesis or solid phase peptide synthesis as described, for example, in Chapter 9 entitled "Peptide Synthesis" by Atherton and Shephard which is included in a publication entitled "Synthetic Vaccines" edited by Nicholson and published by Blackwell Scientific Publications. Alternatively, peptides can be produced by digestion of a polypeptide of the invention with proteinases such as endoLys-C, endoArg-C, endoGlu-C and staphylococcus V8-protease. The digested fragments can be purified by, for example, reverse phase high performance liquid chromatographic (RP-HPLC) techniques. A biologically active fragment according to the invention can therefore be the substrate of a transglutaminase, such as mammalian, e.g., human Factor XIIIa, as long as it is capable of being immobilized onto components of the extracellular matrix (ECM), or to transglutaminase substrates e.g. an alpha-2 plasmin inhibitor (a2PI)-derived Q-peptide or a polypeptide that comprises the target sequence for FXIIIa, e.g., a non-natural, for example, a chimeric/fusion polypeptide that in addition to the target sequence comprises further polypeptidic sequence parts that may serve desired biological functions, which may be the adhesion to biological or non-biological materials.

As used herein, the terms "effective amount" when used with reference to a composition of an active compound refers to an amount or dosage sufficient to produce a desired result (e.g., for therapy with the compositions of the present invention). In the case of sustained delivery compositions comprising compounds of the invention, the desired result may be a desired reduction in inflammation and/or an increase in the healing of a lesioned tissues as detectable by visual inspection of newly formed tissue, closing of lesions, absence or reduction of swelling and/or pain, reduced wound size, etc.), for example. More specifically, a "therapeutically effective amount" of an active compound of the invention, is an amount of that particular compound which is sufficient to inhibit, or halt altogether, or ameliorate, heal, alleviate or cure, for some desired period of time, one or more clinically defined pathological processes associated with the condition at issue. The effective amount may vary depending on the specific active compound(s) selected, and a variety of other factors and conditions related to the subject to be treated and the severity of the conditions, e.g., the age, weight and health of the patient as well as dose response curves and toxicity data obtained in preclinical animal work would be among those considered. If the compound(s) is to be contacted with the cells *in vitro,* one would also design a variety of pre-clinical in vitro studies to assess such parameters as uptake, half-life, dose, toxicity, etc. The determination of an effective amount or a therapeutically effective amount for a given agent is well within the ability of those skilled in the art.

In the context of treating or preventing a condition or achieving an desired biological effect in systems in vitro the use or administration of an effective amount of active to an individual in need of such treatment or prophylaxis or to the in vitro cell system, either in a single dose or as part of a series, that is effective for treatment or prophylaxis of a condition. The effective amount will vary depending upon the type of environment (e.g., the type and size of lesion, tissue type(s), etc.) and upon the physical condition of the individual to be treated, the formulation of the composition comprising the herein disclosed compounds, the assessment of the medical situation, and other relevant factors.

By "therapeutically effective amount or dose" or "sufficient amount or dose" herein is meant a dose that produces effects for which it is administered. The exact dose will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

The terms "pharmaceutically effective", "therapeutically effective", "pharmaceutically active", or "therapeutically active" means that a synthetic compound of the invention so described is determined to have activity that affects a medical parameter or disease state (for example, the size of and/or inflammation and/or pain associated with a tissue lesion).

"Patient" as that term is used herein, refers to the recipient of the treatment. In a specific embodiment, the patient is a mammal, such as a human, canine, murine, feline, bovine, ovine, swine or caprine. In a preferred embodiment, the patient is a human.

Sustained delivery compositions of the present invention are particularly useful for slow release of active agents with short biological half-lives, such as certain macromolecules such as proteins and peptides. As a result, the sustained delivery compositions described herein may also enable the use of alternative routes of administration when the sustained delivery compositions include a therapeutic drug and are administered to a patient for slow release or targeted delivery of the drug to the site requiring therapy. Frequently, therapeutic use of growth factors such as IGF-I has focused on application in soluble forms. Thus, they are rapidly cleared from the treated area after administration thereby restricting therapy due to safety issues and cost effectiveness. The *in situ* generated delivery system for IGF-I facilitates local IGF-I accumulation and enables spatiotemporal release of the growth factor controlled by natural ECM remodeling processes. Thus, IGF-I is highly effective at very low dose and allows proper tissue morphogenesis with very low toxicity by emulating the natural storage mechanism.

As used herein, the terms "function" or "functional activity" refer to a biological, e.g., enzymatic function.

By "isolated" is meant material that is substantially or essentially free or purified from components that normally accompany it in its native state. For example, the compound according to the invention may be modified subsequent to isolation from their natural or laboratory-produced environment, or they may be used in isolated form in vitro, or as components of devices, compositions, etc.

By "obtained from" is meant that a sample such as, for example, a polypeptide is isolated from, or derived from, a particular source of the host or cells cultured in vitro. For example, the extract can be obtained from a tissue or a biological fluid sample isolated directly from the host. Therefore, the compounds of the present invention may be recombinantly produced or obtained from biological sources and be purified before further use in vitro and/or in vivo.

By "pharmaceutically acceptable carrier" is meant a solid or liquid filler, stabilizer, diluent or encapsulating substance that can be safely used in administration routes when applied to an animal, e.g. a mammal, including humans.

"Therapeutic treatment", and "treatment", refers any type of therapy referred to herein, e.g., treatment of lesions, including the treatment to prevent the deterioration or worsening of such lesions or wounds, or treatment of chronic diseases like rheumatoid arthritis or tendinitis, or for post-operative prevention of inflammation or joint destruction.

The terms "lesions" or "wounds" are understood as any pathologic, inflammatory, painful, exogenously or endogenously caused disturbance of the integrity of a tissue of an organism, e.g., through any disease, surgical intervention, accident (cuts, stabs, burns, etc.), (auto)inflammation, and the like.

The terms "identical" or percent "identity," in the context of two or more polypeptide sequences, refer to two or more sequences or subsequences that are the same or have a specified percentage of amino acid residues that are the same (i.e., 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection (see, e.g., NCBI web site http://www.ncbi.nlm.nih.gov/BLAST/ or the like). Such sequences are then said to be "substantially identical." The definition also includes sequences that have deletions and/or additions, as well as those that have substitutions. As described below, the preferred algorithms can account for gaps and the like. Preferably, identity exists over a region that is, for example, at least about 4, 5, 6, 7, 8, amino acids in length.

For sequence comparisons of compounds disclosed herein, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

The terms "polypeptide," "peptide" and "protein" are used interchangeably herein to refer to a polymer of amino acid residues. The terms apply also to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid. Amino acids may be referred to herein by either their commonly known three letter symbols or by the one-letter symbols recommended by the IUPAC-IUB Biochemical Nomenclature Commission.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" or "derivative" where the alteration results in the substitution of an amino acid, e.g., with a chemically similar amino acid. Conservative substitution tables providing functionally similar amino acids are well known in the art. According to the present invention, modified variants of the compounds of the invention retain the activity of acting as a donor for transglutaminases, preferably FXIIIa, and may be immobilized on a suitable substrate to which it may be crosslinked enzymatically. The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (Creighton, Proteins (1984)).

Below, embodiments of the invention are disclosed. When the expression "and/or" is used, this means that each member of a respective list may be analyzed or used individually, or that more than one member of said list may be used. Further, when a list of members is combined with another list or lists of members, this means that each and every possible combination is encompassed by the present invention even if not every combination of the lists is explicitly, i.e. literally disclosed.

### Embodiments of the invention

Subject matter of the invention is a synthetic compound suitable for transglutaminase-mediated incorporation or coupling of a therapeutic or diagnostic molecule into the extracellular matrix (attachment to extracellular matrix components or synthetic ECM-derived materials, such as fibrin or collagen matrices, hyaluronic acid gels or elastin networks serving as targets for transglutaminases, preferably human transglutaminases (e.g. FXIIIa)), wherein said synthetic compound comprises
(a) at least one anchor domain and
(b) at least one therapeutic or diagnostic molecule,
wherein said anchor domain is selected from the group comprising:
i. the D domain of Insulin Growth Factor-1 (IGF-I) as depicted in SEQ ID NO: 1,
ii. a derivative of i) having at least 50% identity with SEQ ID NO: 1,
iii. a fragment of i) or ii), wherein said fragment comprises at least the four C-terminal amino acid residues depicted in SEQ ID NO: 1, or a fragment comprising the at least five C-terminal amino acid residues depicted in SEQ ID NO: 1, or a fragment comprising the at least six C-terminal amino acid residues depicted in SEQ ID NO: 1, or a fragment comprising the at least seven C-terminal amino acid residues depicted in SEQ ID NO: 1, and
wherein a therapeutic or diagnostic molecule is directly or indirectly linked to any of the anchor domains referred to in i) to iii).

As used herein, the term extracellular matrix component comprises, e.g., fibronectin, osteopontin, decorin, collagen, hyaluronic acid, elastin, as well as mixtures thereof, e.g., in form of matrices, gels, networks, spray-dried or otherwise bound components, such as fibronectin that is attached to a support, etc.

In embodiments of the invention, the derivatives and/or fragments maintain the lysine residue at position 6 of SEQ ID NO: 1. Some embodiments of such derivatives and/or fragments are depicted in the Sequence Listing in SEQ ID Nos: 2 to 10.

In context of the present application, directly linked or indirectly linked refers to peptidic linkages or peptide bonds, covalently binding, etc. Suitable conjugation or linking methods include covalent or non-covalent (such as biotin-(strept)avidin systems or heparin binding domains), preferably site-specific bioconjugation strategies. The coupling can be performed by chemical or enzymatic bio-orthogonal approaches.

Subject matter of the present invention is also a synthetic compound according to the previous embodiment for use as therapeutic agent or diagnostic agent.

Subject matter of the present invention is also a synthetic compound according to any one of the preceding embodiments, wherein said therapeutic agent comprises a growth factor (e.g. IGF-1, FGF2, VEGF, bone morphogenetic protein(s), e.g., BMP-2), a therapeutically active polypeptide, anti-apoptotic molecules, anti-inflammatory molecules, antibiotics, hormones, antibodies, immune modulating cytokines (Interleukins, e.g. IL-2, IL-4; Interferons, e.g. INFα2a). In the context of the present invention, the term "therapeutic treatment", relates to the amelioration of the respective underlying disease or symptom and comprises the alleviation of such disease or symptom, the reduction of undesirable disease symptoms, the improvement of health, the prevention from worsening or the occurrence of a disease or symptom in a patient in need thereof being subjected to the treatment.

Further, in context of the present invention, the synthetic compound as defined above in none of the herein described embodiments has the amino acid sequence of wild-type (wt) IGF-I. The synthetic compound may therefore be designated as a non-wt IGF-I compound. An inventive synthetic compound may, however, comprise an anchoring domain that does not correspond to the entire sequence depicted in SEQ ID NO: 1, e.g., a fragment thereof or a derivative thereof, linked to one or more of the domains A, B, and C of IGF-1 that are involved in IGF-1R binding and activation and/or binding of IGFBPs.

Subject matter of the present invention is also a synthetic compound according to any one of the preceding embodiments, wherein said diagnostic agent comprises a fluorescent moiety or another detectable moiety, e.g. a chromogenic compound, a radioactive compound, etc. Fluorescent moieties and other detectable moieties can be selected from those known to a person skilled in the art, e.g., those available on the market from companies such as Thermo Fisher, Sigma Aldrich, etc.

Subject matter of the present invention is also a synthetic compound according to any one of the preceding embodiments, further comprising a cleavable linker. The cleavable linker may be selected from those responding to elevated protease concentrations during disease onset or progression (e.g., matrix metalloproteases, in case of inflammatory conditions) or to changes in external stimuli, such as reduction in pH or elevated H₂O₂ concentrations [18, 19].

Subject matter of the present invention is therefore also a synthetic compound according to any one of the preceding embodiments, wherein said compound further comprises a cleavable linker between the anchor domain and the therapeutic or diagnostic molecule. The cleavable linker may be derived from collagen type I, or may be any linker molecule that can be cleaved in a desired environment of use or administration of the herein described inventive products (synthetic compounds, pharmaceutical or diagnostic compositions/formulations, devices as defined below).

Subject matter of the present invention is also a pharmaceutical or diagnostic composition or formulation comprising a synthetic compound as defined in any of the preceding embodiments.

Subject matter of the present invention is also a pharmaceutical or diagnostic composition or formulation comprising a synthetic compound as defined in any of the preceding embodiments, wherein said composition or formulation is suitable for localized administration.

Subject matter of the present invention is also a synthetic compound as defined in any of the preceding embodiments for use in the treatment of lesions and/or tissue regeneration in lesioned locations and/or prevention of inflammation in lesions, rheumatoid arthritis, osteoarthritis, bone fractures, tendinitis, heart disease, atherosclerosis and impaired wound healing, bacterial infection, muscle wasting diseases, amongst other pathological states.

Subject matter of the present invention is also a synthetic compound as defined in any of the preceding embodiments in combination with an enzyme or fragment having transglutaminase activity, preferably a mammalian transglutaminase selected from the group comprising of FXIII, TG1, TG2, TG3, TG4, TG5, TG6 and TG7, more preferably human Factor Xllla, for use in the treatment of lesions and/or tissue regeneration in lesioned locations and/or prevention of inflammation in lesions, rheumatoid arthritis, osteoarthritis, bone fractures, tendinitis, heart disease, atherosclerosis and impaired wound healing, bacterial infection, muscle wasting disease amongst other pathological states.

Subject matter of the present invention is also the co-delivery of the herein described compounds or compositions together with FXIII and fibrinogen to achieve gel formation *in situ* so that therapeutic or diagnostic moieties referred to herein may be bound or immobilized to or onto such gel.

Subject matter of the present invention is also a synthetic compound or the pharmaceutical composition or formulation according to any of the above embodiments as defined in any of the preceding embodiments for use in the treatment of lesions and/or tissue regeneration in lesioned locations and/or prevention of inflammation in lesions bone fractures, tendinitis, heart disease, atherosclerosis and impaired wound healing, amongst other pathological states, wherein said composition or formulation is suitable for the localized administration, wherein the localized administration is preferably selected from the group of topical administration, including transdermal, ophthalmic, nasal, otologic, enteral, pulmonal and urogenital administration or local or systemic injection, including subcutaneous, intra-articular, intravenous, intracardiac, intramuscular, intraosseous or intraperitoneal administration.

Subject matter of the present invention is also a synthetic compound as defined in any of the preceding embodiments or the pharmaceutical composition or formulation according to any of the above embodiments for use in the treatment of lesions and/or tissue regeneration in lesioned locations and/or prevention of inflammation in lesions, bone fractures, tendinitis, heart disease, atherosclerosis and impaired wound healing, amongst other pathological states, wherein said composition or formulation is suitable for the localized administration, wherein the localized administration is preferably selected from the group comprising topical administration, administration to the site of a lesion, administration to the site of surgery, wherein, in addition to the synthetic compound of the invention as defined above, a transglutaminase is either separately or simultaneously administered. Therefore, a transglutaminase as described herein may be a component of the pharmaceutical or diagnostic composition or formulations of the invention or it may be administered as component of a separate formulation or composition.

Subject matter of the present invention is also a device comprising a synthetic compound or pharmaceutical composition or a pharmaceutical formulation as defined in any of the preceding embodiments. The device can incorporate any of the compounds as defined in the preceding embodiments, together with a transglutaminase to provide sustained release of the compounds and the crosslinking enzyme.

Subject matter of the present invention is also a device according to the previous embodiment, wherein the device is suitable as a delivery system for immediate and/or sustained release of a compound as defined in any one of the preceding claims. The devices according to the present invention may therefore also be used as drug delivery systems or controlled drug release systems.

Subject matter of the present invention is also a device according to the preceding embodiments, wherein said device is selected from the group comprising patches, artificially produced tissues, implants, scaffolds, porous vascular grafts, stents, wound dressings composed of a biocompatible fleece (preferably made of hydrocolloids, polyacrylate, alginate, hydrogels, or foams), artificially produced tissues, bone-replacements, polymer networks, hydrogels (preferably composed of fibrin, collagen, elastin, hyaluronic acid or silk proteins).

Subject matter of the present invention is also an in vitro method of tissue engineering comprising the steps of
- providing an extracellular matrix substrate comprising a specific amino acid sequence serving as a target for a transglutaminase, preferably FXIIIa
- providing a synthetic compound as defined in any of the preceding claims,
- exposing the extracellular matrix substrate and the compound as defined in steps (i) and (ii) to an enzyme having transglutaminase activity under conditions and in a medium suitable for transamination.

The extracellular matrix substrate is preferably a target for human FXIIIa and may be fibronectin or a suitable derivative or fragment thereof that maintains its activity of serving as a target for the immobilization of the synthetic compounds as defined herein. The target may be modified with a glutamine substrate, such as NQEQVSPL (SEQ ID NO: 11).

Subject matter of the present invention is also an artificial tissue obtainable by a method according to the preceding embodiment.

Subject matter of the present invention is also an artificial tissue according to the preceding embodiment for use in the treatment of lesions, wounds, diseases or conditions requiring tissue replacements selected from the group comprising surgically treated tissues, tendinitis, rheumatoid arthritis, osteoarthritis, bone replacements, tooth replacements, etc.

Subject matter of the present invention is also a method of incorporating exogenous peptides into hydrogels (preferably composed of fibrin, collagen, elastin, hyaluronic acid or silk proteins) using the herein described synthetic compounds.

Subject matter of the present application is a synthetic compound as defined in the preceding embodiments for use in the treatment of lesions and/or tissue regeneration and/or prevention of inflammation, wherein said compound comprises at least one anchoring domain that is selected from the group comprising:
i) the D domain of Insulin Growth Factor-1 (IGF-I) as depicted in SEQ ID NO: 1,
ii) a derivative of i) having at least 50% identity with SEQ ID NO: 1, wherein the derivative maintains the capability of acting as a substrate for transglutaminases, e.g., (human) FXIIIa, and can be immobilized on a suitable substrate or matrix and preferably consists of the four C-terminal amino acids of SEQ ID NO: 1 (AKSA); the derivative preferably has at least 60% identity with SEQ ID NO: 1, or at least 70% identity with SEQ ID NO: 1, or at least 75% identity with SEQ ID NO: 1, or at least 80% identity with SEQ ID NO: 1, or at least 85% identity with SEQ ID NO: 1, or at least 90% identity with SEQ ID NO: 1, or at least 95% identity with SEQ ID NO: 1;
iii) a fragment of i) or ii), wherein said fragment comprises at least the four C-terminal amino acid residues depicted in SEQ ID NO: 1, or a fragment comprising the at least five, or a fragment comprising the at least six, or a fragment comprising the at least seven amino acids of SEQ ID NO: 1, or a derivate thereof having at least 75% identity, at least 80% identity, or at least 85% to the amino acid sequence depicted in SEQ ID NO: 1, wherein said fragments comprise at least the four C-terminal amino acid residues depicted in SEQ ID NO: 1.

As used herein said fragment as part of the synthetic compound described herein may be incorporated in medicaments, compositions (liquid formulations suitable for injection into diseased tissue, solid formulations including lyophilized or spray-dried formulations for reconstitution before parenteral administration, patches, gels, salves, ointments, endoscopically administrable compositions for local or system release, e.g. stents, artificial bones (bone replacements), skin, etc.), or devices that are used for any functional activities underlying the purposes indicated herein, in particular in the treatment of lesions, or in vitro into compositions or media used for tissue engineering purposes.

The synthetic compound may be a chimeric (fusion) polypeptide comprising any of the above synthetic compounds i) to iii) and at least one (cleavable) linker peptide and/or a further therapeutically or diagnostically active polypeptide sequence. As used herein, a "chimeric polypeptide" or a "fusion polypeptide" designates any polypeptide that comprises the D domain of IGF as defined above or a derivative or fragment thereof that maintains the capacity of being recognized and immobilized on or incorporated into a suitable substrate or matrix using a transglutaminase (e.g., FXIIIa, preferably human FXIIIa), wherein said polypeptide is chemically bound (e.g., with a peptide bond) or otherwise linked to a heterologous peptide sequence, which means that it is not bound to the adjacent polypeptide found in natural (e.g., wild-type) IGF-I molecules neighboring the D domain of said protein; the heterologous polypeptide may be derived from any polypeptide of interest, e.g., growth factors, hormones, therapeutically active peptides, particularly peptides involved in the regeneration, repair and growth of tissues or cells, but the heterologous peptides may also be artificial sequences that have a desired function, e.g., acting as a spacer or a binding site for an antibody or for any other molecule of interest, for example, a linker molecule that can be cleaved to release a polypeptide or other molecule having a desired function, e.g., being involved in the regeneration, repair and growth of tissues or cells. Instead of a heterologous polypeptide, the anchor domain as defined above may also be linked to or bound to a diagnostic molecule as defined above.

Subject-matter of the invention is also a synthetic compound for use in the treatment of lesions and/or tissue regeneration according to the above embodiments, wherein the treatment of lesions and/or tissue regeneration is for post-surgical lesions, on skin lesions, tendon lesions complications, and arthritic lesions. As used herein, "lesion complications" refers to a condition, wherein the healing process is not progressing as intended or is reversed, or wherein an infection or inflammation or any undesirable immune reaction occurs, e.g. accompanied by non-intended apoptosis or necrosis, fever, and so forth.

The synthetic compound may be any of the above specified synthetic compounds, in particular those having the desired functional activity, which includes pharmaceutically or therapeutically or diagnostically effective or active polypeptides or other molecules for therapeutic use or diagnostic molecules as defined above. The synthetic compounds use for such treatments are used at therapeutically or diagnostically effective doses / amounts.

Subject-matter of the invention is also a synthetic compound product as defined in any of the foregoing embodiments, particularly, in the above embodiments relating to the synthetic compounds for use in the treatment of lesions and/or tissue regeneration and/or prevention of inflammation.

Subject-matter of the invention is also a pharmaceutical composition/formulation as defined in any of the foregoing embodiments, further comprising an enzyme having transglutaminase activity. In some embodiments, the enzyme may be an activated transglutaminase, such as Factor XIIIa (preferably human FXIIIa) or tissue transglutaminase, or the enzyme may be an inactive transglutaminase that is activated upon administration by thrombin. As FXIIIa requires the presence of calcium as cofactor, exogenous calcium sources may also be included into the inventive compositions or formulations, e.g., CaCl₂.

Subject-matter of the invention is also a device comprising a pharmaceutical composition or a pharmaceutical formulation as defined in any of the foregoing embodiments. A device may take any form that is suitable to deliver the synthetic compounds or any one of the compositions or formulations of the present invention. It may comprise biological and/or synthetic materials and may take form of a patch, a stent, an implantable device, an artificially produced tissue (which may be obtainable by means of tissue engineering), an artificial bone or ankle, soluble components of ECM or synthetic biomaterials, hydrogels or components able to be cross-linked in situ catalyzed by transglutaminase (e.g. fibrin) etc.

Subject-matter of the invention is also a device as defined in any of the foregoing embodiments, wherein the device is a delivery system for immediate and/or sustained release of the synthetic compound as defined in any of the foregoing embodiments.

Subject-matter of the invention is also an in vitro method of angiogenesis comprising the steps of
(i) providing a(n) (extracellular) matrix substrate comprising a specific amino acid sequence serving as a target for human transglutaminase FXIIIa,
(ii) providing a synthetic compound as defined in any of the foregoing embodiments, wherein a therapeutically active molecule is known to be involved in angiogenesis, e.g., VEGF or other angiogenic factors or derivatives, e.g., mutant forms of such therapeutically active molecules,
(iii) exposing the (extracellular) matrix substrate and the compound as defined in steps (i) and (ii) to an enzyme having transglutaminase activity under conditions and in a medium suitable for transamination.

Subject-matter of the invention is also a method of treatment of an individual in need thereof and/or the amelioration of and/or the prevention of deterioration of a disease in an individual in need thereof, e.g., in a patient having a lesion or wound or catabolic/atrophic condition as defined in any one of the preceding embodiments, by administration to said individual of a therapeutically efficient amount of any of the synthetic compounds and/or pharmaceutical compositions as defined above.

The administration of the compounds according to this invention and pharmaceutical compositions according to the invention may be performed in any of the generally accepted modes of administration available in the art. Illustrative examples of suitable modes of administration include intravenous, oral, nasal, inhalable, parenteral, topical, transdermal and rectal delivery. Parenteral and intravenous delivery forms are preferred. In aspects of the invention injectable compositions comprising a therapeutically effective amount of the compounds of the invention are provided, including salts, esters, isomers, solvates, hydrates and polymorphs thereof, at least one vehicle comprising water, aqueous solvents, organic solvents, hydro-alcoholic solvents, oily substances, or mixtures thereof, and optionally one or more pharmaceutically acceptable excipients. Standard knowledge regarding these pharmaceutical ingredients and pharmaceutical formulations/compositions may be found, *inter alia,* in the 'Handbook of Pharmaceutical Excipients'; Edited by Raymond C Rowe, Paul J Sheskey, Walter G Cook and Marian E Fenton; May 2012 and/or in Remington: The Science and Practice of Pharmacy, 19th edition. The pharmaceutical compositions may be formulated in the form of a dosage form for oral, intravenous, nasal, inhalable, parenteral, topical, transdermal and rectal and may thus comprise further pharmaceutically acceptable excipients, such as buffers, solvents, preservatives, disintegrants, stabilizers, carriers, diluents, fillers, binders, lubricants, glidants, colorants, pigments, taste masking agents, sweeteners, flavorants, plasticizers, and any acceptable auxiliary substances such as absorption enhancers, penetration enhancers, surfactants, co-surfactants, and specialized oils.

The proper excipient(s) is (are) selected based in part on the dosage form, the intended mode of administration, the intended release rate, and manufacturing reliability. Examples of common types of excipients include also various polymers, waxes, calcium phosphates, sugars, etc.

Polymers include cellulose and cellulose derivatives such as HPMC, hydroxypropyl cellulose, hydroxyethyl cellulose, microcrystalline cellulose, carboxymethylcellulose, sodium carboxymethylcellulose, calcium carboxymethylcellulose, and ethylcellulose; polyvinylpyrrolidones; polyethylenoxides; polyalkylene glycols such as polyethylene glycol and polypropylene glycol; and polyacrylic acids including their copolymers and crosslinked polymers thereof, e.g., Eudragit^{®}(Rohm), polycarbophil, and chitosan polymers. Waxes include white beeswax, microcrystalline wax, carnauba wax, hydrogenated castor oil, glyceryl behenate, glycerylpalmitol stearate, and saturated polyglycolyzed glycerate. Calcium phosphates include dibasic calcium phosphate, anhydrous dibasic calcium phosphate, and tribasic calcium phosphate. Sugars include simple sugars, such as lactose, maltose, mannitol, fructose, sorbitol, saccharose, xylitol, isomaltose, and glucose, as well as complex sugars (polysaccharides), such as maltodextrin, amylodextrin, starches, and modified starches.

The pharmaceutical compositions of the present invention may be formulated into various types of dosage forms, for instance as solutions or suspensions, or as tablets, capsules, granules, pellets or sachets for oral administration. A particularly preferred pharmaceutical composition is in the form of a solid oral dosage form, preferably tablets. The tablet is preferably a tablet for swallowing. It may optionally be coated with a film coat comprising, in essence, any suitable inert coating material known in the art. The above lists of excipients and forms are not exhaustive.

The pharmaceutical composition of the present invention can be manufactured according to standard methods known in the art. Granulates according to the invention can be obtained by dry compaction or wet granulation. These granulates can subsequently be mixed with e.g. suitable disintegrating agents, glidants and lubricants and the mixture can be compressed into tablets or filled into sachets or capsules of suitable size. Tablets can also be obtained by direct compression of a suitable powder mixture, i.e. without any preceding granulation of the excipients. Suitable powder or granulate mixtures according to the invention are also obtainable by spray drying, lyophilization, melt extrusion, pellet layering, coating of the active pharmaceutical ingredient or any other suitable method. The so obtained powders or granulates can be mixed with one or more suitable ingredients and the resulting mixtures can be delivered in sterile primary packaging devices for reconstitution before parenteral administration Injectable compositions of the present invention may contain a buffer (for example, sodium dihydrogen phosphate, disodium hydrogen phosphate and the like), an isotonizing agent (for example, glucose, sodium chloride and the like), a stabilizer (for example, sodium hydrogen sulfite and the like), a soothing agent (for example, glucose, benzyl alcohol, mepivacaine hydrochloride, xylocaine hydrochloride, procaine hydrochloride, carbocaine hydrochloride and the like), a preservative (for example, p-oxybenzoic acid ester such as methyl p-oxybenzoate and the like, thimerosal, chlorobutanol, benzyl alcohol and the like) and the like, if necessary. In addition, the injectable composition of the present invention may contain vitamins and the like. Further, injectable compositions of the present invention may contain an aqueous solvent, if necessary. Examples of the aqueous solvent include purified water for injection, physiological saline solution, and glucose solution. In injectable compositions of the present invention, the pharmaceutical compound may be solid. As used herein, the "solid" comprises crystals and amorphous substances which have conventional meanings. The form of the solid component is not particularly limited, but powder is preferred in view of dissolution rate.

### Pharmaceutical compositions

Still another aspect of the present invention relates to the use of the compound according to claim 1 as an active ingredient, together with at least one pharmaceutically acceptable carrier, excipient and/or diluents for the manufacture of a pharmaceutical composition for the treatment and/or prophylaxis of lesions or wounds or musculoskeletal disorders. Musculoskeletal disorders (MSDs) are conditions that can affect muscles, bones, and joints. They include conditions such as tendinitis, carpal tunnel syndrome, osteoarthritis, rheumatoid arthritis, fibromyalgia and bone fractures.

Such pharmaceutical compositions comprise the peptide as an ECM anchor, together with an active ingredient and at least one pharmaceutically acceptable buffer, carrier, excipient, diluents, preservatives or the like. The pharmaceutical compositions of the present invention can be prepared in a conventional solid or liquid carrier or diluents and a conventional pharmaceutically-made adjuvant at suitable dosage level in a known way. Preferably the compound is suitable for intravenous administration or suitable for topical administration or suitable for administration by inhalation.

Administration forms include, for example, pills, tablets, film tablets, coated tablets, capsules, liposomal formulations, micro- and nano-formulations, powders and deposits. Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain the compounds according to the present invention.

In some embodiments of the invention, local administration methods of the compounds and/or compositions disclosed herein are preferred.

The compounds of the invention can also be administered in form of its pharmaceutically active salts. Suitable pharmaceutically active salts comprise acid addition salts and alkali or earth alkali salts. For instance, sodium, potassium, lithium, magnesium or calcium salts can be obtained.

The pharmaceutical compositions according to the present invention will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are hydrogels, elixirs, dispersible granules, syrups, suspensions, creams, lotions, solutions, emulsions, suspensions, dispersions, and the like. Suitable dosage forms for sustained release include tablets having layers of varying disintegration rates or controlled release polymeric matrices delivered with the active components. The pharmaceutical compositions may be comprised of 1 to 95% by weight of the compounds of the invention.

As pharmaceutically acceptable carrier, excipient and/or diluents can be used HSA, lactose, sucrose, cellulose, mannitol.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below. Additionally, the compositions of the present invention may be formulated in sustained release form to provide the rate controlled release of any one or more of the components or active ingredients to optimize the therapeutic effects. Suitable dosage forms for sustained release include controlled release polymeric matrices or hydrogels embedding the active components. Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations which are intended to be converted, shortly before use, to liquid form preparations for parenteral administration. Such liquid forms include solutions, suspensions and emulsions.

The compounds of the present invention may also be deliverable transdermally. The transdermal compositions may take the form of creams, lotions, aerosols and/or emulsions and can be included in a transdermal patch of the matrix or reservoir type as are conventional in the art for this purpose.

The transdermal formulation of the compounds of the invention is understood to increase the bioavailability of said compound into the circulating blood. One problem in the administration of peptidic drugs in general is the loss of bioactivity due to the formation of insolubles in aqueous environments or due to degradation. Therefore, stabilization of compounds for maintaining their fluidity and maintaining their biological activity upon administration to the patients in need thereof needs to be achieved. Prior efforts to provide active agents for medication include incorporating the medication in a polymeric matrix whereby the active ingredient is released into the systemic circulation. Known sustained-release delivery means of active agents are disclosed, for example, in US4235988, US4188373, US4100271, US447471, US4474752, US4474753, or US4478822 relating to polymeric pharmaceutical vehicles for delivery of pharmaceutically active chemical materials to mucous membranes. The pharmaceutical carriers are aqueous solutions of certain polyoxyethylene-polyoxypropylene condensates. These polymeric pharmaceutical vehicles are described as providing for increased drug absorption by the mucous membrane and prolonged drug action by a factor of two or more. The substituents are block copolymers of polyoxypropylene and polyoxyethylene used for stabilization of drugs such as insulin.

Aqueous solutions of polyoxyethylene-polyoxypropylene block copolymers (poloxamers) are useful as stabilizers for the compounds. Aside from serving as a stabilizer for the compound, poloxamers provide excellent vehicles for the delivery of the compound, and they are physiologically acceptable. Poloxamers, also known by the trade name Pluronics (e.g. Pluronic F127, Pluronic P85, Pluronic F68) have surfactant properties that make them useful in industrial applications. Among other things, they can be used to increase the water solubility of hydrophobic, oily substances or otherwise increase the miscibility of two substances with different hydrophobicities. For this reason, these polymers are commonly used in industrial applications, cosmetics, and pharmaceuticals. They have also been used as model systems for drug delivery applications. In situ gelation of pharmaceutical compositions based on poloxamer that are biologically triggered are known in the art (e.g. US5256396), describing compositions containing poloxamer 407 and water at specified concentrations.

Gels refer to the active ingredients dispersed or solubilized in a hydrophilic semi-solid matrix. Powders for constitution refer to powder blends containing the active ingredients and suitable diluents which can be suspended in water and may contain optionally buffer salts, lactose, amino acids, excipients, sugars and isotonisation reagents.

Recently, increasingly improved and potent protein-based and peptide-based drugs have been developed by the biotech industry. However, the prophylactic and/or therapeutic use of many other protein- or peptide-based compounds has been hampered because of their susceptibility to proteolytic breakdown, rapid plasma clearance, peculiar dose-response curves, immunogenicity, bioincompatibility, and/or the tendency of peptides and proteins to undergo aggregation, adsorption, and/or denaturation. These characteristics often render traditional methods of drug delivery ineffective or sub-optimal when applied to protein or peptide based drugs. Therefore, an immense amount of interest has been increasingly placed on controlled and/or sustained release drug delivery systems to maintain the therapeutic efficacy or diagnostic value of these important classes of biologically active agents. One of the primary goals of sustained delivery systems is to maintain the levels of an active agent within an effective range and ideally at a constant level. One approach for sustained delivery of an active agent is by microencapsulation, in which the active agent is enclosed within a polymeric matrix. The importance of biocompatible and/or biodegradable polymers as carriers for parenteral drug delivery systems is now well established. Biocompatible, biodegradable, and relatively inert substances such as poly(lactide) (PLA) or poly(lactide-co-glycolide) (PLG) structures such as microparticles or films containing the active agent to be administered are commonly employed sustained delivery devices (for review, see M. Chasin, Biodegradable polymers for controlled drug delivery. In: J.O. Hollinger Editor, Biomedical Applications of Synthetic Biodegradable Polymers CRC, Boca Raton, FL (1995), pp. 1-15; T. Hayashi, Biodegradable polymers for biomedical uses. Prog. Polym. Sci. 19 4 (1994), pp. 663-700; and Harjit Tamber, Pal Johansen, Hans P. Merkle and Bruno Gander, Formulation aspects of biodegradable polymeric microspheres for antigen delivery Advanced Drug Delivery Reviews, Volume 57, Issue 3, 10 January 2005, Pages 357-376). A relatively steady release of one or more active agents incorporated within such polymers is possible because of the degradation profile of these polymers in an aqueous environment. By encapsulating active agents in a polymer matrix in various forms such as microparticles and/or films the active agent is released at a relatively slow rate over a prolonged time. Achieving sustained drug release in such a manner may afford less frequent administration, thereby increasing patient compliance and reducing discomfort; protection of the therapeutic compound within the body; potentially optimized prophylactic or therapeutic responses and prolonged efficacy; and avoidance of peak-related side-effects by maintaining more-constant blood levels of the active agent. Furthermore, these compositions can oftentimes be administered by injection, allowing for localized delivery and high local concentrations of the active agents.

With regard to highly active biologics, such as growth factors, local in the form of a bolus injection results in rapid diffusion from the region of interest and can cause severe side effects and limit efficacy. The oldest way is to use biophysical retention by changing the biophysical properties in form of viscosity, porosity, hydrophobicity or charge of the material to attain a purposeful delivery. This strategy often substantially modifies the properties of the tissue and conditions for cells, requiring more appropriate, biocompatible release mechanisms. Novel methods follow a more precise and regulated path to deliver low dose of bioactive substances by using the natural ability of certain growth factors to interact with ECM glycoproteins e.g. heparin binding sites of FGF or using ECM glycoproteins or ECM fragments. The natural retention of growth factors in the ECM provides a method of controlled release, triggered by the disease itself and not external factors.

### Methods of treatment

As discussed above, the present invention provides a method of treatment of a disease, in particular wound lesions, tendinitis, osteoarthitis or other therapies benefitting from the anabolic activity of IGF-I. Treatment includes any of amelioration, alleviation, prevention from worsening, and curing a disease such as defined above, e.g., a wound or lesion of any tissue, e.g. a surgical wound, an (auto-) inflamed site of the patient's body, etc.. Other diseases may be also be treated with different therapeutically active compounds.

Treatment methods of the invention comprise the step of administering to a subject a therapeutically effective amount of a synthetic compound according to the invention or a pharmaceutical composition of the invention. The administration may be effected by any route, e.g., dermally, parenterally, topically, etc.

In some embodiments, local administration methods of the synthetic compounds and/or compositions disclosed herein are preferred.

As indicated previously "therapeutically effective amount" of a synthetic compound according to the invention preferably refers to the amount necessary to achieve the therapeutic outcome.

The choice of the optimal dosage regime and duration of medication, particularly the optimal dose and manner of administration of the active compounds necessary in each case can be determined by a person skilled in the art on the basis of his/her expert knowledge.

Subject matter of the present invention is also any of the above the above synthetic compounds in method of manufacturing a medicament for the treatment of any of the above mentioned conditions or diseases.

As defined above, the pharmaceutical compositions, formulations or medicaments for administration to an individual in need thereof may, as further component, comprise a transglutaminase. Alternatively, a composition, formulation or medicament comprising a transglutaminase may be administered separately.

### Tissue engineering methods

Subject matter of the present invention is also a method of tissue engineering using the compounds disclosed herein, wherein these compounds are substrates subjected to a transglutaminase reaction, thereby immobilizing the substrates to a material, e.g. another type of substrate, a matrix (such as naturally derived or synthetic ECM or ECM components), which may comprise biomolecule and/or synthetic molecules that act as scaffold for the immobilization. Tissue engineering scaffolds made of microporous scaffolds containing nanofibrous, nanoporous hydrogels formed from self-assembling peptides. These scaffolds provide a template on which cells can migrate, divide, secrete new matrix and differentiate. Typical tissue engineering scaffolds are porous and can be categorized as having pores on either a micrometer scale, i.e. microporous, or a nanometer scale, i.e. nanoporous. Scaffolds having pores on a micrometer scale, or having average pore diameter of about 10 to 1000 microns, are composed of a variety of biocompatible materials including metals, ceramics and polymers. Such scaffolds include solid-cast structures, open-pore foams, felts, meshes, nonwovens, woven and knitted constructs. The mechanical and conformational properties can be chosen by composition of the material and the design of the scaffold. Desirable mechanical properties include the ability to be sutured in place and good handling strength.

Composition, design and construction of the scaffold are also important to how tissue responds to the scaffold. The scaffold can be shaped to maximize surface area, to allow adequate diffusion of nutrients and growth factors to cells present in or growing into it. For example, the maximum distance over which adequate diffusion through densely packed cells can occur is in the range of about 100 to 300 microns, under conditions similar to those that occur in the body, wherein nutrients and oxygen diffuse from blood vessels moving into the surrounding tissue. Taking these parameters into consideration, one of skill in the art would configure a scaffold having pores on a micrometer scale as having sufficient surface area for the cells to be nourished by diffusion until new blood vessels interdigitate the implanted scaffold.

Scaffolds having pores on a nanometer scale, e.g. having average pore diameter of about 10 nanometers to 1 micron, are often composed of hydrogels. A hydrogel is a substance formed when a natural or synthetic organic polymer is cross-linked via covalent, ionic or hydrogen bonds to create a three-dimensional open-lattice structure, which entraps water molecules and forms a gel. Examples of materials that can be used to form a hydrogel include polyamides, methylcellulose, collagen, extracellular matrix (ECM), polysaccharides such as alginate, polyphosphazines, polyacrylates which are crosslinked tonically, high molecular weight poly(oxyalkylene ether) block copolymers such as those sold under the tradename PLURONCIS (BASF Corp., Mount Olive, N.J.), nonionic polymerized alkylene oxide compounds such as those sold under the tradename TETRONCIS (BASF Corp., Mount Olive, N.J.), or polyethylene oxide-polypropylene glycol block copolymers which are crosslinked by temperature or pH, respectively.

Hydrogels provide conformable, malleable, or injectable materials for administering cells into a tissue. They do not, however, have mechanical integrity. Synthetic hydrogels can be sterilized and do not have the risk of associated infectious agents. However, most synthetic hydrogels do not mimic the extracellular matrix and therefore do not direct cellular growth or function. Hydrogels of natural extracellular matrix are biocompatible and can mimic the native cellular environment. However, natural hydrogels, unless made from autologous material, may elicit an immune response and may have associated infectious agents. Natural hydrogels, such as EHS mouse sarcoma basement membrane, or fibrin, have a fiber diameter of about 5 to about 10 nanometers, water content of about 80 to about 97 weight percent and average pore diameter of about 50 to about 400 nanometers.

The present invention relates to tissue-engineering scaffolds comprising a microporous scaffold comprising a biocompatible material suitable for use in tissue-engineering scaffolds and a nanofibrous, nanoporous hydrogel formed at least partially from, or supported in growth and development by the compounds of the present invention. At least a portion of the hydrogel is disposed within the pores of the microporous scaffold, thus providing tissue-engineering scaffolds having average pore diameters in the nanometer range and that provide both mechanical properties suitable for implantation into a body of a mammal and excellent tissue response once implanted in the body. The materials used to form the microporous scaffold and the nanofibrous, nanoporous hydrogel may have similar or different degradation times and may be seeded with cells or contain bioactive compounds. The bioactive synthetic compounds of the present invention can be immobilized on the scaffold or on the components of the hydrogel catalyzed by transglutaminase to provide a controlled release as a function of protease activity in contrast to the unspecific adsorption and diffusion of conventional approaches.

### Examples section

### EXAMPLE 1

### Crosslinking efficiency of the D domain derived from human and mouse IGF-I and truncated versions thereof using a glutamine substrate derived from α-2 plasmin inhibitor catalyzed by FXIIIa

Using solid phase peptide synthesis, the isolated peptide sequence of the D domain of human IGF-I (SEQ ID NO: 1) and mouse IGF-I (SEQ ID NO: 6) were synthesized and purified using preparative HPLC to achieve > 95% purity. Additionally, a collection of N-terminally truncated versions of both sequences were created, in order to test if 7 or less amino acids are sufficient to be recognized by Factor Xllla. According to a trypsin digest of coupled IGF-I, only K68 is modified and therefore, a reduction of the sequence to the last 3 amino acids could be possible. The truncated versions were manufactured and purified accordingly. Lyophilized peptides were dissolved in Tris buffer (20 mM Tris-HCl, 150 mM NaCl, pH 7.6) and reacted with a glutamine substrate (SEQ ID NO: 13, derived from α-2 plasmin inhibitor) in presence of 0.1 M calcium chloride and 10 U/ml Factor XIIIa (Fibrogammin^{®}, activated with 0.02 U/ml thrombin). Following 30 minutes incubation at 37 °C, the coupling reaction was stopped and the amount of crosslinked peptide analyzed using analytical RP-HPLC. The crosslinking ratios using equimolar amounts of both peptides (D domain and glutamine acceptor) are depicted in Table 1.

**TABLE 1: Crosslinking efficacy of the D domain and N-terminally truncated versions derived from human (SEQ ID NO: 1-5) and mouse (SEQ ID NO: 6-10) IGF-I, respectively.**

| **SEQ ID NO:** | **SEQUENCE** | **Crosslinking Efficacy (1:1 molar ratio)*** |
|---|---|---|
| **SEQ ID NO: 1** | PLKPAKSA | 82,94 ± 0,79 |
| **SEQ ID NO: 2** | LKPAKSA | 72,45 ± 1,57 |
| **SEQ ID NO: 3** | KPAKSA | 58,37 ± 2,10 |
| **SEQ ID NO: 4** | PAKSA | 44,18 ± 3,23 |
| **SEQ ID NO: 5** | AKSA | 49,52 ± 1,29 |
| **SEQ ID No: 5 (-AS 67)** | KSA | 21,40 ± 1,96 |
| **SEQ ID NO: 6** | PLKPTKAA | 84.16 ± 7.76 |
| **SEQ ID NO: 7** | LKPTKAA | 69.42 ± 8.62 |
| **SEQ ID NO: 8** | KPTKAA | 55.68 ± 3.25 |
| **SEQ ID NO: 9** | PTKAA | 33.69 ± 1.41 |
| **SEQ ID NO: 10** | TKAA | 39.26 ± 4.07 |
| **SEQ ID NO: 10 (-AS 67)** | KAA | 24.68 ± 3.42 |

| | | |
|---|---|---|
| *Crosslinking to glutamine substrate sequence (SEQ ID NO: 11) derived from α2PI, incubation for 30 minutes with 10 U/ml FXIIIa; Sequences SEQ ID NO: 5 (-AS 67) and 10 (-AS 67) lack one additional C-terminal amino acid residue, i.e., amino acid residue 67 of the wild-type IGF-1 proteins of humans and mice, respectively. | | |

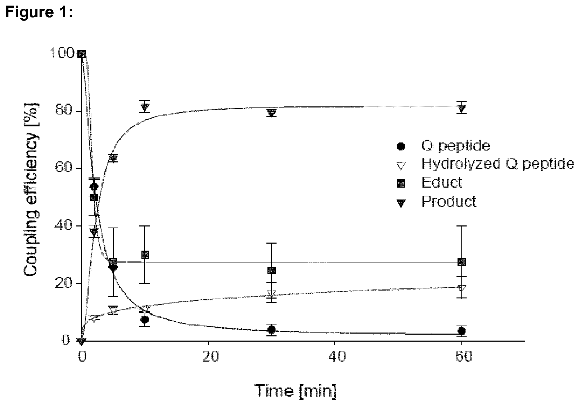

From the results summarized in Table 1, the isolated D domains of human (SEQ ID NO: 1) and mouse IGF-I (SEQ ID NO: 6) show comparable coupling efficacy, resulting in > 80% covalent conjugation after 30 minutes. Apparently, no exact structure of both adjacent amino acids to K68 is required to be recognized and crosslinked by Factor XIIIa. An N-terminal reduction of the sequence decreases, but does not abolish, the crosslinking rate. Even the peptide sequences reduced to the 3 C-terminal amino acids yielded about 20 % crosslinked product. In general, coupling rate increases with increased chain length and the complete D domain (AS 63-70) demonstrated the highest crosslinking ratio (Table 1). The time course of the coupling reaction between the Q peptide and the D domain (SEQ ID NO: 1) confirmed, that the crosslinking is very fast, achieving about 80% conversion within 10 minutes (Figure 1).

**TABLE 2: Crosslinking efficacy as a function of the molar ratio between the human D domain of IGF-I (SEQ ID NO: 1) and the Glutamine substrate (derived from α2PI)**

| **Molar excess of the glutamine sequence** | **Crosslinking Efficacy (1:1 molar ratio)*** |
|---|---|
| **0.5** | 44.48 ± 2.45 |
| **1** | 81.06 ± 5.13 |
| **2,5** | 84.67 ± 2.02 |
| **3.75** | 87.53 ± 5.33 |
| **5** | 84.91 ± 6.23 |
| **10** | 88.67 ± 6.52 |

### EXAMPLE 2

### Modification of the isolated D domain of IGF-I with small molecules and immobilization of the conjugate on fibronectin and ECM using FXIIIa

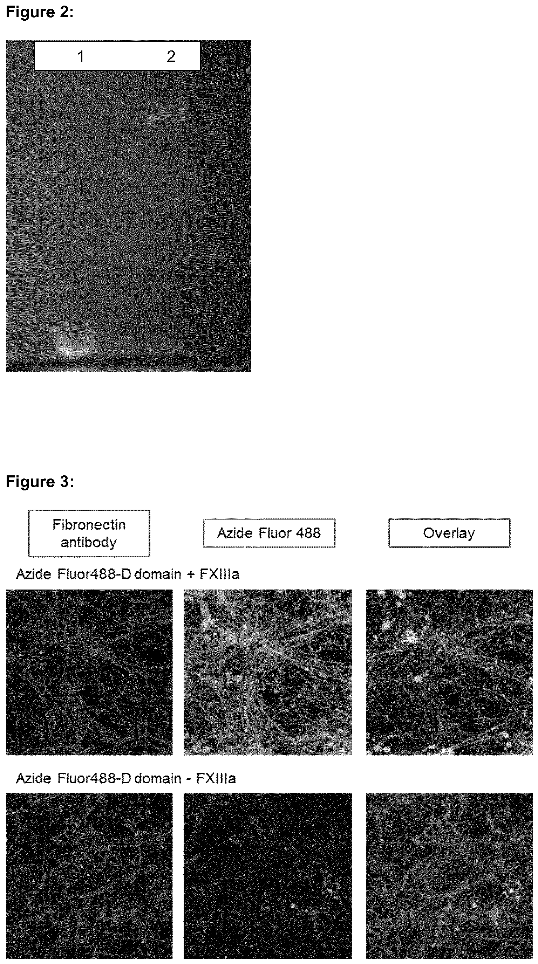

As proof of concept (for covalent attachment of a bioactive factor to extracellular matrix (ECM), we modified the D domain of IGF-I (SEQ ID NO: 1) with an dibenzocyclooctyne group (DBCO) at the N-terminus (SEQ: DBCO-PLKPAKSA), followed by coupling of a small molecule (fluorescent dye Azide Fluor 488) onto cell derived, isolated ECM. The ECM was produced by NIH3T3 fibroblasts and on day 6 decellularized and washed to remove cell debris and adsorbed proteins. Taking advantage of the intrinsic property of fibronectin, representing a glutamine donor for Factor XIII, we immobilized the fluorescent dye-D domain conjugate on the N-terminus of fibronectin without impairment of ECM structure and integrity. According to previous studies, FXIIIa shows specificity for glutamine residue #3 of fibronectin and this Q3 represents the main target for conjugations [16]. Labelling of fibronectin at the N-terminus was found to not preclude cellular recognition of Fn conjugates or abolish Fn-Fn interactions that are essential for fibril formation [17] [14]. We immobilized Alexa488-D domain on ECM with high efficacy (Figure 2) and confirmed the co-localization with fibronectin using confocal laser scanning microscopy (Figure 3).

This example shows that different molecules can be synthetically coupled to the peptide sequence for incorporation into the ECM. As the peptide sequence can be modified in many different ways without losing the affinity for crosslinking by Factor XIIIa, any biologically or medically useful molecule or macromolecule can be coupled to the ECM or synthetic matrices by transglutaminase.

### EXAMPLE 3

### In situ immobilization of IGF-I on decellularized ECM using FxIIIa

Due to the intrinsic transglutaminase recognition sequence of native IGF-I, Factor Xllla mediated linkage provides an easy, site-specific and efficient method for creation of IGF-I conjugates or for bioorthogonal immobilization of the growth factor on biological surfaces.

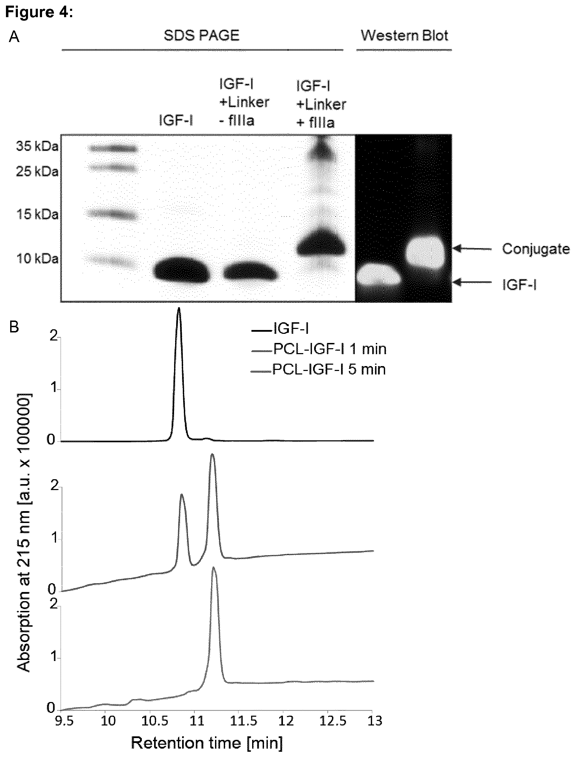

The specificity and efficiency of the reaction was analyzed and compared to the isolated D domain (SEQ ID NO: 1) using a linker containing the glutamine substrate sequence derived from α-2 plasmin inhibitor and analyzed by SDS-PAGE and by western blotting using a monoclonal anti-IGF-I antibody (Figure 4A). The transamidation was carried out at 37°C for 5 min in Tris buffer (20 mM Tris-HCL, 150 mM sodium chloride, pH 7.6) with 0.1 M calcium chloride in presence of factor XIIIa (Fibrogammin^{®}, activated with 0.02 U/ml thrombin). The resulting conjugate was analyzed by MALDI-MS and the site-specific conjugation confirmed. Analysis of reaction kinetics by HPLC confirms that the crosslinking of the complete protein is also exceptionally fast and efficient (Figure 4B) yielding high concentrations of crosslinked conjugate after the short incubation time.

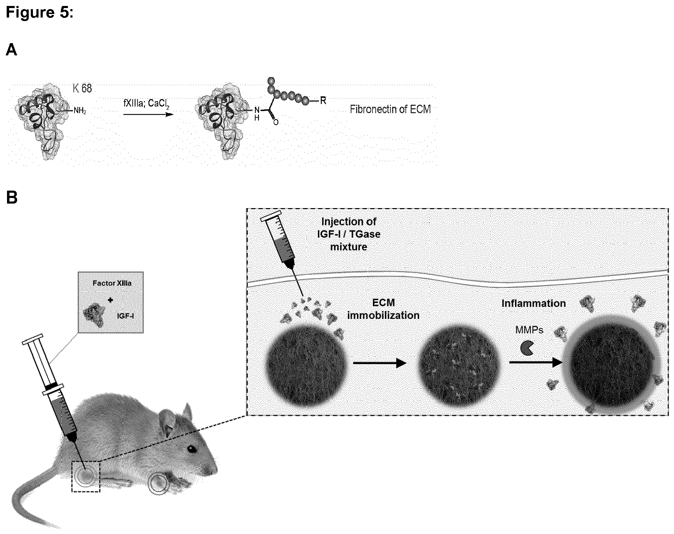

Besides the efficient synthesis of IGF-I conjugates, the growth factor can be applied locally, either as a therapy of diseases like rheumatoid arthritis or tendinitis, or for post-operative prevention of inflammation or joint destruction. By administering the growth factor together with the crosslinking enzyme in one syringe, either during the surgery into the open wound or into the injured joint, the diseased tissue can be supported in the recovery process (Figure 5). Locally, the transglutaminase catalyzes the crosslinking of IGF-I with endogenous fibronectin of the ECM, resulting in local accumulation of the anti-apoptotic growth factor (Figure 5A). If a post-operative inflammation in the joint occurs, accompanied by invasion of macrophages and release of MMPs, the growth factor is released and instantly inhibits the flaring inflammation and prevents apoptosis of cartilage and muscle cells (Figure 5B).

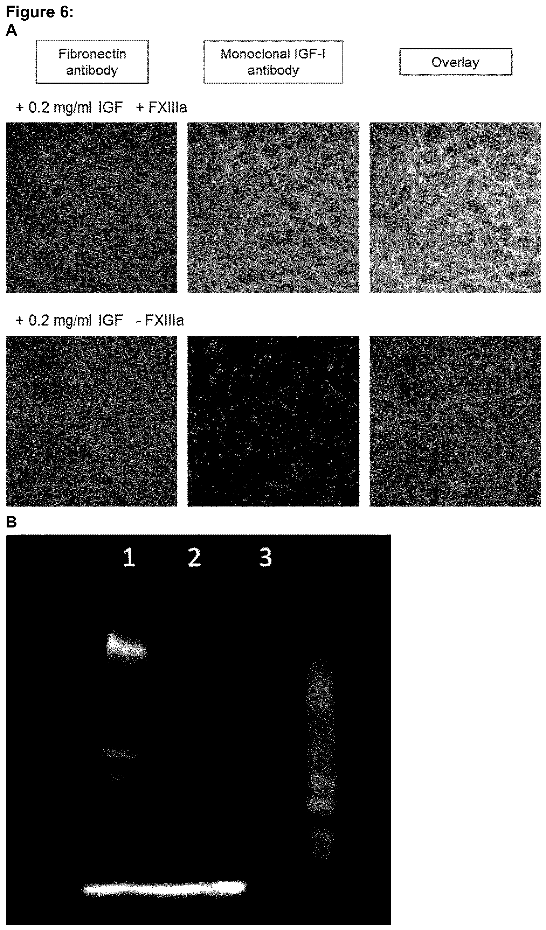

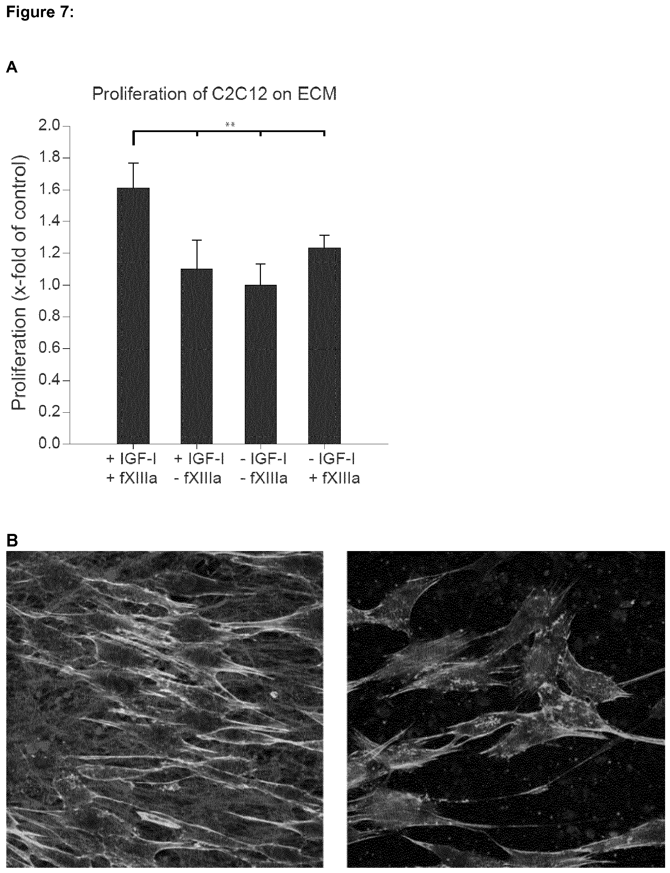

To test this strategy, native IGF-I was immobilized onto fibroblast derived, isolated extracellular matrix in presence of Factor Xllla (Figure 6). Conjugation of IGF-I to the ECM with subsequent antibody staining confirmed the co-localization with fibronectin (Figure 6A). The transamidation reaction was carried out at 37°C for 10 min in Tris buffer at pH 7.6 and is suitable for performance under physiological conditions. To prove that the transamidation reaction is specific for fibronectin, we performed a western blot with soluble fibronectin and IGF-I and confirmed the conjugation in presence of activated transglutaminase (Figure 6B). Bioactivity of immobilized IGF-I was confirmed by promoting cell proliferation of mouse myoblast cells (Figure 7A, B).

### EXAMPLE 4

### Immobilization of proteins modified with IGF-I's D domain on fibronectin and ECM using Factor Xllla

The isolated D domain of human IGF-I (SEQ ID NO: 1) can also be inserted into any other peptide or protein structure (such as growth factors or other bioactive macromolecules) to promote retention in the ECM. To this end, local accumulation and storage of these biomolecules in the ECM is facilitated, according to nature's developed strategy for IGF-I. Modification of peptides, proteins or biomaterial surfaces with this sequence enables the site-specific and efficient conjugation without the need for protein modification such as insertion of unnatural amino acids.

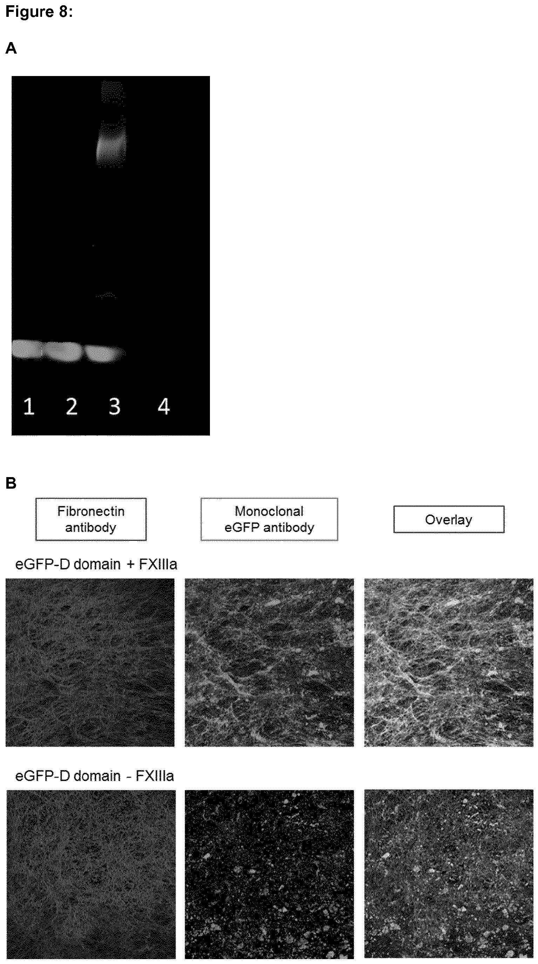

The same coupling reaction as described for IGF-I (EXAMPLE 3) was also performed with enhanced green fluorescent protein (eGFP), previously modified with the D domain of human IGF-I (SEQ ID NO: 1), and immobilized using factor XIIIa (Figure 8). The western blot using plasma fibronectin and the eGFP-D domain conjugate showed effective conjugation (Figure 8A), and also the immobilization of the modified protein on ECM was confirmed (Figure 8B).

### Description of the figures

**Figure 1****:** Coupling efficiency as a function of incubation time using the D domain of human IGF-I (SEQ ID NO: 1) reacted with a glutamine substrate sequence (SEQ ID NO: 11) in presence of Factor Xllla.
**Figure 2****:** Immobilization of the covalent conjugate of the D domain (SEQ ID NO: 1) and the small molecule Azide Fluor 488 on fibronectin (isolated from human plasma) catalyzed by transglutaminase. The conjugates coupled to fibronectin in absence (lane #1) and presence (lane #2) of Factor Xllla were transferred to a 5% SDS-PAGE gel and analyzed for fluorescence.
**Figure 3****:** Confocal laser scanning microscopy images of the Azide Fluor 488-D domain conjugate immobilized on isolated ECM in presence (panel 1) or absence (panel 2) of factor Xllla. The green fluorescence of the immobilized dye-peptide conjugate co-localized with fibronectin (red fluorescence).
**Figure 4****:** (A) SDS PAGE and immunoblotting of human IGF-I after incubation with the glutamine substrate peptide modelled from the N-terminus of alpha-2 plasmin inhibitor (SEQ ID NO: 11) attached to a protease cleavable linker (PCL) in absence and presence of FXIIIa. (B) HPLC analysis of IGF-I coupling kinetics mediated by fxIIIa.
**Figure 5****:** (A) Schematic illustration of site-directed coupling of IGF-I to the ECM protein fibronectin mediated by human Factor XIIIa. (B) *In situ* immobilization of IGF-I on ECM by co-injection of IGF-I and factor XIIIa into the joint.
**Figure 6****:** (A) Confocal laser scanning microscopy images of IGF-I immobilized on isolated ECM. The immobilized IGF-I was visualized with a monoclonal IGF-I antibody (green) und co-localized with fibronectin (red). (B) Western Blot of human IGF-I after incubation with fibronectin in presence (lane 1) and absence (lane 2) of factor Xllla with unreacted IGF-I as control (lane 3).
**Figure 7****:** Bioactivity of ECM-immobilized IGF-I. (A) Native IGF-I was coupled to ECM in presence/absence of factor Xllla, followed by seeding of C2C12 myoblasts on this naturally derived matrix and cell proliferation was measured after 48 hours incubation using WST-1 (Data are shown as mean ± STDEV, n = 3; ** p < 0.01). (B) The cells were fixed, stained with actin (green) and DAPI (cell nuclei, blue) and ECM-immobilized IGF-I was visualized using a monoclonal IGF-I antibody (red).
**Figure 8****:** (A) Western Blot of enhanced green fluorescent protein modified with the D domain (SEQ ID NO: 1) (eGFP-D domain, lane #1) after incubation with fibronectin in absence (lane #2) and presence (lane #3) of Factor XIIIa. (B) Confocal laser scanning microscopy images of eGFP modified with the D domain (SEQ ID NO: 1) and immobilized on isolated ECM in presence (panel 1) or absence (panel 2) of factor XIIIa. The immobilized eGFP was visualized with a monoclonal eGFP antibody (green) und co-localized with fibronectin (red).

### References

[1] K. Lee, E.A. Silva, D.J. Mooney, Growth factor delivery-based tissue engineering: general approaches and a review of recent developments, Journal of The Royal Society Interface, 8 (2011) 153-170.
[2] M.M. Martino, P.S. Briquez, E. Güç, F. Tortelli, W.W. Kilarski, S. Metzger, J.J. Rice, G.A. Kuhn, R. Müller, M.A. Swartz, J.A. Hubbell, Growth Factors Engineered for Super-Affinity to the Extracellular Matrix Enhance Tissue Healing, Science, 343 (2014) 885-888.
[3] I. Schultz, J. Wurzel, L. Meinel, Drug delivery of Insulin-like growth factor I, European Journal of Pharmaceutics and Biopharmaceutics, 97, Part B (2015) 329-337.
[4] E.M. Agency, Increlex EPAR - Scientifc Discussion, in, London, 2007.
[5] L. Meinel, O.E. Illi, J. Zapf, M. Malfanti, H.P. Merkle, B. Gander, Stabilizing insulin-like growth factor-I in poly(D,L-lactide-co-glycolide) microspheres, J Control Release, 70 (2001) 193-202.
[6] M. Singh, B. Shirley, K. Bajwa, E. Samara, M. Hora, D. O'Hagan, Controlled release of recombinant insulin-like growth factor from a novel formulation of polylactide-co-glycolide microparticles, J Control Release, 70 (2001) 21-28.
[7] X.M. Lam, E.T. Duenas, A.L. Daugherty, N. Levin, J.L. Cleland, Sustained release of recombinant human insulin-like growth factor-I for treatment of diabetes, J Control Release, 67 (2000) 281-292.
[8] A. Denley, L.J. Cosgrove, G.W. Booker, J.C. Wallace, B.E. Forbes, Molecular interactions of the IGF system, Cytokine & Growth Factor Reviews, 16 (2005) 421-439.
[9] M.A. Cascieri, G.G. Chicchi, J. Applebaum, N.S. Hayes, B.G. Green, M.L. Bayne, Mutants of human insulin-like growth factor I with reduced affinity for the type 1 insulin-like growth factor receptor, Biochemistry, 27 (1988) 3229-3233.
[10] T. Sitar, G.M. Popowicz, I. Siwanowicz, R. Huber, T.A. Holak, Structural basis for the inhibition of insulin-like growth factors by insulin-like growth factor-binding proteins, Proceedings of the National Academy of Sciences, 103 (2006) 13028-13033.
[11] M. Sivaramakrishnan, T.I. Croll, R. Gupta, D. Stupar, D.R. Van Lonkhuyzen, Z. Upton, G.K. Shooter, Lysine residues of IGF-I are substrates for transglutaminases and modulate downstream IGF-I signalling, Biochimica et Biophysica Acta (BBA) - Molecular Cell Research, 1833 (2013) 3176-3185.
[12] J.C. Schense, J.A. Hubbell, Cross-linking exogenous bifunctional peptides into fibrin gels with factor XIIIa, Bioconjugate Chemistry, 10 (1999) 75-81.
[13] T.J. Sanborn, P.B. Messersmith, A.E. Barron, In situ crosslinking of a biomimetic peptide-PEG hydrogel via thermally triggered activation of factor XIII, Biomaterials, 23 (2002) 2703-2710.
[14] Z.-Y. Zhang, P. Shum, M. Yates, P.B. Messersmith, D.H. Thompson, Formation of Fibrinogen-Based Hydrogels Using Phototriggerable Diplasmalogen Liposomes, Bioconjugate Chemistry, 13 (2002) 640-646.
[15] A. Sala, M. Ehrbar, D. Trentin, R.G. Schoenmakers, J. Voros, F.E. Weber, Enzyme Mediated Site-Specific Surface Modification, Langmuir, 26 (2010) 11127-11134.
[16] R.P. McDonagh, J. McDonagh, T.E. Petersen, H.C. Thøgersen, K. Skorstengaard, L. Sottrup-Jensen, S. Magnusson, Amino acid sequence of the factor Xllla acceptor site in bovine plasma fibronectin, FEBS Letters, 127 (1981) 174-178.
[17] S.M. Früh, P.R. Spycher, M. Mitsi, M.A. Burkhardt, V. Vogel, I. Schoen, Functional Modification of Fibronectin by N-Terminal FXIIIa-Mediated Transamidation, ChemBioChem, 15 (2014) 1481-1486.
[18] A.C. Braun, M. Gutmann, R. Ebert, F. Jakob, H. Gieseler, T. Lühmann, L. Meinel, Matrix Metalloproteinase Responsive Delivery of Myostatin Inhibitors, Pharmaceutical Research, (2016).
[19] T. Lühmann, L. Meinel, Nanotransporters for drug delivery, Current Opinion in Biotechnology, 39 (2016) 35-40.
[20] D.J. Fieten, The Effect of a Myostatin Antagonist on the Healing of Burn Wounds in Skin, in, University of Waikato, Hamilton, New Zealand, 2009.
[21] J. Ritzer, T. Lühmann, C. Rode, M. Pein-Hackelbusch, I. Immohr, U. Schedler, T. Thiele, S. Stübinger, B.v. Rechenberg, J. Waser-Althaus, F. Schlottig, M. Merli, H. Dawe, M. Karpíšek, R. Wyrwa, M. Schnabelrauch, L. Meinel, Diagnosing peri-implant disease using the tongue as a 24/7 detector, Nature Communications, 8 (2017) 264.
[22] C.L. Nikolajsen, T.F. Dyrlund, E.T. Poulsen, J.J. Enghild, C. Scavenius, Coagulation Factor Xllla Substrates in Human Plasma: IDENTIFICATION AND INCORPORATION INTO THE CLOT, The Journal of Biological Chemistry, 289 (2014) 6526-6534.
[23] B.-H. Hu, P.B. Messersmith, Rational Design of Transglutaminase Substrate Peptides for Rapid Enzymatic Formation of Hydrogels, Journal of the American Chemical Society, 125 (2003) 14298-14299.

## Claims

1. A synthetic compound suitable for transglutaminase-mediated incorporation of a therapeutic or diagnostic molecule into an extracellular matrix or a synthetic extracellular matrix component, wherein said compound comprises
(a) at least one anchor domain and
(b) at least one therapeutic or diagnostic molecule,
wherein said anchor domain is selected from the group comprising:
i. the D domain of Insulin Growth Factor-1 (IGF-I) as depicted in SEQ ID NO: 1,
ii. a derivative of i) having at least 50% identity with SEQ ID NO: 1,
iii. a fragment of i) or ii), wherein said fragment comprises at least the four C-terminal amino acid residues depicted in SEQ ID NO: 1, or a fragment comprising the at least five, or a fragment comprising the at least six, or a fragment comprising the at least seven, or a derivate thereof having at least 75% identity to the amino acid sequences depicted in SEQ ID NO: 1, and
wherein a therapeutic or diagnostic molecule is directly or indirectly linked to any of the anchor domains referred to in i) to iii).

2. The synthetic compound according to claim 1 for use as therapeutic agent or diagnostic agent.

3. The synthetic compound according to claim 1 and 2, wherein said therapeutic agent comprises a growth factor, a therapeutically active polypeptide, an antibody, a polymer, a small molecule, or combinations thereof.

4. The synthetic compound according to claim 1 and 2, wherein said diagnostic agent comprises a fluorescent moiety, a radiolabeled tracer or mass tag.

5. The synthetic compound according to any of one of claims 1 to 4, wherein said compound further comprises a cleavable linker between the anchor domain and the therapeutic or diagnostic molecule.

6. A pharmaceutical or diagnostic composition or formulation comprising a synthetic compound as defined in any of the preceding claims.

7. The pharmaceutical or diagnostic composition or formulation according to any of the preceding claims, wherein said composition or formulation is suitable for the localized administration.

8. A synthetic compound as defined in any of the preceding claims for use in the treatment or diagnosis of a disease selected from the group comprising lesions and/or tissue regeneration in lesioned locations and/or prevention of inflammation in lesions, rheumatoid arthritis, osteoarthritis, musculoskeletal diseases, bone fractures, tendinitis, heart disease, atherosclerosis and impaired wound healing, amongst other pathological states, and/or bacterial infections.

9. The synthetic compound as defined in any of the preceding claims or a pharmaceutical or diagnostic composition or formulation according to any of the preceding claims for use in the treatment or diagnosis of a disease selected from the group comprising lesions and/or tissue regeneration in lesioned locations and/or prevention of inflammation in lesions, rheumatoid arthritis, osteoarthritis, musculoskeletal diseases, bone fractures, tendinitis, heart disease, atherosclerosis and impaired wound healing, and/or bacterial infections in combination with an enzyme or fragment having transglutaminase activity, preferably a mammalian transglutaminase selected from the group consisting of FXIII, TG1, TG2, TG3, TG4, TG5, TG6 and TG7, more preferably human Factor XIIIa.

10. The synthetic compound as defined in any of the preceding claims or a pharmaceutical or diagnostic composition or formulation according to any of the preceding claims for use in the treatment or diagnosis of a disease selected from the group comprising lesions and/or tissue regeneration in lesioned locations and/or prevention of inflammation in lesions, rheumatoid arthritis, osteoarthritis, musculoskeletal diseases, bone fractures, tendinitis, heart disease, atherosclerosis and impaired wound healing, and/or bacterial infections, wherein said synthetic compound or pharmaceutical or diagnostic composition or formulation is suitable for the localized administration, wherein the localized administration is preferably selected from the group of topical administration, including transdermal, ophthalmic, nasal, otologic, enteral, pulmonal and urogenital administration or,local or systemic injection, including subcutaneous, intra-articular, intravenous, intracardiac, intramuscular, intraosseous or intraperitoneal administration.

11. A device comprising a synthetic compound or pharmaceutical or diagnostic composition or a pharmaceutical or diagnostic formulation as defined in any of the preceding claims.

12. The device according to claim 11, wherein the device is suitable as a delivery system for immediate and/or sustained release of a compound as defined in any one of the preceding claims.

13. The device according to claim 12, wherein said device is selected from the group comprising patches, implants, scaffolds, porous vascular grafts, stents, and/or wound dressings composed of a biocompatible fleece, preferably made of hydrocolloids, polyacrylate, alginate, hydrogels, or foams, and/or artificially produced tissues, bone-replacements, polymer networks, hydrogels, preferably composed of fibrin, collagen, elastin, hyaluronic acid or silk proteins.

14. An in vitro method of tissue engineering comprising the steps:
(i) providing an extracellular matrix substrate comprising a specific amino acid sequence serving as a target for transglutaminase,
(ii) providing a synthetic compound as defined in any of the preceding claims,
(iii) exposing the extracellular matrix substrate and the compound as defined in steps (i) and (ii) to an enzyme having transglutaminase activity under conditions and in a medium suitable for transamidation.

15. An artificial tissue obtainable by a method according to claim 14.

16. The artificial tissue according to claim 15 for use in the treatment of lesions, wounds, diseases or conditions requiring tissue replacements selected from the group comprising surgically treated tissues, tendinitis, rheumatoid arthritis, bone replacements, tooth replacements, chronic wounds.


SEQUENCE LISTING

<110>  Julius-Maximilians-Universität Würzburg

<120>  Pharmaceuticals and devices for the immobilization of IGF-1's D
       domain and derivatives thereof to extracellular matrices by human
        transglutaminase

<130>  U30797EP

<160>  12

<170>  PatentIn version 3.5

<210>  1
<211>  8
<212>  PRT
<213>  Homo sapiens

<400>  1

Pro Leu Lys Pro Ala Lys Ser Ala
1               5


<210>  2
<211>  7
<212>  PRT
<213>  Homo sapiens

<400>  2

Leu Lys Pro Ala Lys Ser Ala
1               5


<210>  3
<211>  6
<212>  PRT
<213>  Homo sapiens

<400>  3

Lys Pro Ala Lys Ser Ala
1               5


<210>  4
<211>  5
<212>  PRT
<213>  Homo sapiens

<400>  4

Pro Ala Lys Ser Ala
1               5


<210>  5
<211>  4
<212>  PRT
<213>  Homo sapiens

<400>  5

Ala Lys Ser Ala
1


<210>  6
<211>  8
<212>  PRT
<213>  Mus musculus

<400>  6

Pro Leu Lys Pro Thr Lys Ala Ala
1               5


<210>  7
<211>  7
<212>  PRT
<213>  Mus musculus

<400>  7

Leu Lys Pro Thr Lys Ala Ala
1               5


<210>  8
<211>  6
<212>  PRT
<213>  Mus musculus

<400>  8

Lys Pro Thr Lys Ala Ala
1               5


<210>  9
<211>  5
<212>  PRT
<213>  Mus musculus

<400>  9

Pro Thr Lys Ala Ala
1               5


<210>  10
<211>  4
<212>  PRT
<213>  Mus musculus

<400>  10

Thr Lys Ala Ala
1


<210>  11
<211>  8

<212>  PRT
<213>  Homo sapiens

<400>  11

Asn Gln Glu Gln Val Ser Pro Leu
1               5


<210>  12
<211>  8
<212>  PRT
<213>  Synthetic sequence (mutant IGF-I D-Domain)

<400>  12

Pro Leu Lys Pro Ala Arg Ser Ala
1               5